# EUROPEAN PATENT APPLICATION

(11) **EP 4 606 376 A1**
(43) Date of publication of application: **27.08.2025**
(21) Application number: 23879105.7
(22) Date of filing: 17.10.2023
(51) Int. Cl.: A61K 31/495, A61K 31/24, A61K 31/215, A61P 31/12, A61P 31/14

(54) **DRUG COMBINATION COMPRISING NAFAMOSTAT AND K777 AND USE THEREOF**

(30) Priority: 17.10.2022 WO PCT/CN2022/125676
(71) Applicant: ShanghaiTech University, Shanghai 201210 (CN); Guangzhou Laboratory, Guangzhou, Guangdong 510005 (CN); Wuhan Institute Of Virology, Chinese Academy of Sciences, Wuhan, Hubei 430071 (CN)
(72) Inventor: YANG, Haitao, Shanghai 201210 (CN); RAO, Zihe, Shanghai 201210 (CN); WANG, Haofeng, Shanghai 201210 (CN); LIU, Xiaoce, Shanghai 201210 (CN); CHEN, Xinwen, Guangzhou, Guangdong 510005 (CN); XIAO, Gengfu, Wuhan, Hubei 430071 (CN); ZHANG, Leike, Wuhan, Hubei 430071 (CN); YANG, Qi, Guangzhou, Guangdong 510005 (CN); JIANG, Biao, Shanghai 201210 (CN); CHEN, Hongli, Shanghai 201210 (CN); PENG, Wei, Guangzhou, Guangdong 510005 (CN); YANG, Xiuna, Shanghai 201210 (CN)
(74) Representative: Greaves Brewster LLP
(86) International application number: PCT/CN2023/124964
(87) International publication number: WO 2024/083117

(57) **Abstract**

The present invention provides a drug combination comprising Nafamostat and K777 and a use thereof. The pharmaceutical composition comprises active ingredients, i.e., Nafamostat and K777. The pharmaceutical composition of the present invention can show a remarkable synergistic effect in related research on anti-coronavirus, and shows improvement of the effectiveness of more than 10 times in an anti-novel coronavirus test.

## Description

The present application claims the right of the priority of Chinese patent application PCT/CN2022/125676 filed on October 17, 2022. The contents of the above Chinese patent application are incorporated herein by reference in its entirety.

### TECHNICAL FIELD

The present disclosure relates to the technical field of biomedicine, and specifically to a pharmaceutical combination comprising Nafamostat and K777, and a use thereof in the manufacture of a medicament for inhibiting SARS-CoV-2 or for treating/preventing a disease caused by SARS-CoV-2.

### BACKGROUND

Coronaviruses (CoVs) are a class of enveloped, positive-sense, single-stranded RNA viruses that can infect a variety of organisms, including humans, mammals, birds, and rodents, and are capable of cross-species transmission between animals and humans, leading to a variety of chronic or acute infectious diseases.

The entire life cycle of coronaviruses is mainly divided into several steps such as viral cell entry, viral transcription and replication, and assembly and release of viral particles. Proteins that play key roles in these vital processes, such as the viral spike protein (Spike), main protease (Mpro), papain-like protease (PLpro), host angiotensin-converting enzyme 2 (ACE2), serine protease TMPRSS2, and cathepsin L, are ideal targets for developing anti-SARS-CoV-2 therapies. Among these, Spike, ACE2, TMPRSS2, and cathepsin L play important roles in the viral cell entry process. Taking SARS-CoV-2 as an example, its entry into the host requires initial recognition and binding of its spike protein (Spike) with the angiotensin-converting enzyme 2 (ACE2) on the surface of the host cell. After binding to the receptor, the Spike needs to be cleaved by the host protease to complete the conformational change, releasing its fusion peptide to mediate fusion between the viral envelope and the cell membrane (Glowacka, Ilona et al. Evidence that TMPRSS2 activates the severe acute respiratory syndrome coronavirus spike protein for membrane fusion and reduces viral control by the humoral immune response. Journal of virology, (2011)).

The main protease (Mpro) is a cysteine protease essential for viral replication and assembly. It contains 11 enzyme cleavage sites and plays a crucial role during the replication and transcription processes of the coronavirus life cycle. Papain-like protease (PLpro) is an important regulatory protein molecule for the formation of the replicase complex (RC), and it is also critical for viral genome transcription and replication.

Angiotensin-converting enzyme 2 (ACE2) is a zinc metalloprotease present in normal lung tissue, such as type I and type II alveolar epithelial cells. Coronavirus invades the lungs by binding to ACE2 through the S protein. After coronavirus infection, ACE2 levels are downregulated, ultimately leading to increased pulmonary capillary permeability, resulting in pulmonary edema, acute severe lung injury, acute lung failure, etc. Thus, ACE2 not only facilitates viral entry into cells but also exacerbates the condition of the infected.

TMPRSS2 is a type II transmembrane serine protease located on the cell membrane surface, regulated by androgens, and its main recognition cleavage sequence is a single arginine or lysine (AFAR D E, VIVANCO I, HUBERT R S, et al. Catalytic cleavage of the androgen-regulated TMPRSS2 protease results in its secretion by prostate and prostate cancer epithelia. Cancer Res, (2001)). TMPRSS2 plays an important role not only in coronavirus invasion but is also highly associated with multiple diseases, such as influenza virus infection and the occurrence of prostate cancer (WANG Z, WANG Y, ZHANG J, et al. Significance of the TMPRSS2: ERG gene fusion in prostate cancer. Mol Med Rep, (2017); ZMORA P, MOLAU-BLAZEJEWSKA P, BERTRAM S, et al. Non-human primate orthologues of TMPRSS2 cleave and activate the influenza virus hemagglutinin. PLoS One, (2017)). Thus, there is a certain research basis for developing TMPRSS2-based drugs, and since TMPRSS2 is located on the cell membrane surface, small-molecule drugs do not need to enter cells, which makes drug development more advantageous. Cathepsin L is a cysteine protease belonging to the papain family. It has a key amino acid, i.e. cysteine, at its substrate-binding and catalytic sites, which plays a key role in performing its function.

Currently, there are no drugs or vaccines available for SARS-CoV and MERS-CoV, resulting in a lack of effective treatment options. In comparison, the successful development of vaccines and antibodies against SARS-CoV-2 has provided effective means for the prevention and treatment of SARS-CoV-2. However, the continuous emergence of SARS-CoV-2 variant strains poses more severe challenges to the prevention and treatment effects of existing vaccines and antibodies (E. Cameroni et al. Broadly neutralizing antibodies overcome SARS-CoV-2 Omicron antigenic shift. Nature, (2021); L. Liu et al. Striking antibody evasion manifested by the Omicron variant of SARS-CoV-2. Nature, (2021); Y. Cao et al. Omicron escapes the majority of existing SARS-CoV-2 neutralizing antibodies. Nature, (2021); S. Cele et al. Omicron extensively but incompletely escapes Pfizer BNT162b2 neutralization. Nature,

(2021)). Similarly, although a series of small-molecule drugs, represented by Pfizer's Paxlovid, have been approved for marketing, the high mutation rate characteristic of SARS-CoV-2, as an RNA virus, presents a risk of drug resistance during subsequent use of antiviral drugs developed against viral targets. Therefore, it is urgent to develop novel antiviral therapies.

### CONTENT OF THE PRESENT INVENTION

In view of the lack of host-targeted antiviral therapies and the lack of effective broad-spectrum drug treatment options for SARS-CoV-2 in the prior art, the present disclosure provides a pharmaceutical combination comprising Nafamostat and K777, and a use thereof.

Nafamostat (structure formula shown in Formula 1) is an orally administered drug with broad-spectrum serine protease inhibitory activity, which has been approved for marketing in Japan. Its primary indication is acute pancreatitis, and it also serves an anticoagulant role during hemodialysis, coronary artery bypass surgery, and liver resection.

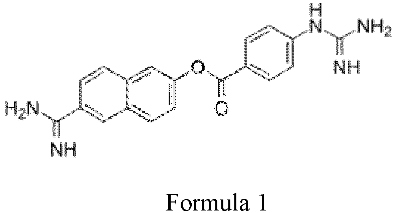

K777 (structure formula shown in Formula 2) is an orally administered irreversible cysteine protease inhibitor. K777 antagonizes a variety of viral infections such as SARS-CoV and Ebola by inhibiting cathepsins B and L. K777 is also an antagonist of the chemokine receptor CCR4.

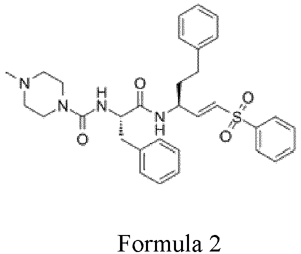

Research has shown that mice with the Cathepsin L and TMPRSS2 genes knocked out did not have significant phenotypic changes compared to wild-type mice, effectively verifying the safety of these host-derived targets (Kim, T.S et al. Phenotypic Analysis of Mice Lacking the Tmprss2-Encoded Protease. Molecular and Cellular Biology, (2006)). Therefore, the technical solution of the present disclosure does not have any potential safety risks.

Since SARS-CoV-2 and its variant can simultaneously conduct Spike cleavage during the invasion of host cells through the proteases Cathepsin L and TMPRSS2, and the extent of dependency on these two ways of cell entry varies depending on host cell type, viral variant strains, and other factors, it is difficult to effectively eliminate viral infections by inhibiting TMPRSS2 or Cathepsin L alone.

Through *in vitro* enzyme activity assays and *in vitro* cell-virus assays, the present disclosure has found that the two-drug combination of Nafamostat and K777 significantly enhances efficacy compared to the use of a single drug. In *in vitro* cell assays against the Delta variant of SARS-CoV-2 , the two-drug combination achieves more than a 10-fold increase in EC₅₀ values, which can be used to treat a disease caused by SARS-CoV-2.

A first aspect of the present disclosure provides a pharmaceutical combination comprising active ingredients Nafamostat and K777.

In some embodiments, the molar ratio of Nafamostat to K777 is 1:0.5 to 1:2.

In some preferred embodiments, the molar ratio of Nafamostat to K777 is 1:1.

In some preferred embodiments, the pharmaceutical combination further comprises a pharmaceutically acceptable carrier or excipient.

In some embodiments, the dosage form of the pharmaceutical combination is selected from one of granules, tablets, pills, capsules, and injections.

A second aspect of the present disclosure provides a use of the pharmaceutical combination according to the first aspect of the present disclosure in the manufacture of a medicament for inhibiting a coronavirus.

In some embodiments, the coronavirus is SARS-CoV-2, SARS-CoV, MERS-CoV, HCoV-HKU1, HCoV-NL63, HCoV-OC43, or HCoV-229E.

In some preferred embodiments, the coronavirus is SARS-CoV-2.

A third aspect of the present disclosure provides a use of the pharmaceutical combination according to the first aspect of the present disclosure in the manufacture of a medicament for treating/preventing a disease caused by a coronavirus.

In some embodiments, the coronavirus is SARS-CoV-2, SARS-CoV, MERS-CoV, HCoV-HKU1, HCoV-NL63, HCoV-OC43, or HCoV-229E.

In some preferred embodiments, the coronavirus is SARS-CoV-2.

A fourth aspect of the present disclosure provides a method for treating/preventing a disease caused by a coronavirus, wherein the method comprises administering the pharmaceutical combination according to the first aspect of the present disclosure or a medicament comprising the pharmaceutical combination to a subject in need thereof.

In some embodiments, the coronavirus is SARS-CoV-2, SARS-CoV, MERS-CoV, HCoV-HKU1, HCoV-NL63, HCoV-OC43, or HCoV-229E.

In some preferred embodiments, the coronavirus is SARS-CoV-2.

A fifth aspect of the present disclosure provides a kit set, wherein the kit set comprises a kit A and a kit B, wherein the main active ingredient of the kit A is Nafamostat, and the main active ingredient of the kit B is K777.

A sixth aspect of the present disclosure provides the pharmaceutical combination according to the first aspect of the present disclosure for use in inhibiting a coronavirus.

In some embodiments, the coronavirus is SARS-CoV-2, SARS-CoV, MERS-CoV, HCoV-HKU1, HCoV-NL63, HCoV-OC43, or HCoV-229E.

In some preferred embodiments, the coronavirus is SARS-CoV-2.

A seventh aspect of the present disclosure provides the pharmaceutical combination according to the first aspect of the present disclosure for use in treating/preventing a disease caused by a coronavirus.

In some embodiments, the coronavirus is SARS-CoV-2, SARS-CoV, MERS-CoV, HCoV-HKU1, HCoV-NL63, HCoV-OC43, or HCoV-229E.

In some preferred embodiments, the coronavirus is SARS-CoV-2.

To provide an effective and broad-spectrum therapeutic regimen for diseases caused by various coronavirus infections, the present disclosure employs a combined strategy of TMPRSS2 and Cathepsin L inhibitors, demonstrating a synergistic effect that significantly enhances efficacy compared to the use of a single drug. The synergistic effect between Nafamostat and K777 completely blocks viral invasion pathways, thereby offering an effective and broad-spectrum drug therapeutic regimen against SARS-CoV-2 and its variant, and also providing an effective and broad-spectrum drug use strategy for the treatment of major infectious diseases caused by SARS-CoV-2.

Advantages of the present disclosure:

(1) The drug combination of Nafamostat and K777 demonstrates significant synergistic effects in the related research of anti-coronavirus, exhibiting a 10-fold or more increase in efficacy in anti-SARS-CoV-2 assays.

(2) The drug safety of Nafamostat and K777 is reliable, and the clinical trials of the two-drug combination can be rapidly advanced, providing an effective and broad-spectrum treatment to combat the SARS-CoV-2 epidemic and its ever-emerging variants.

### BRIEF DESCRIPTION OF THE DRAWINGS

FIG. 1A is a schematic diagram of the inhibitory results of the small molecule Nafamostat on the host protease TMPRSS2; FIG. 1B is a schematic diagram of the inhibitory results of the small molecule K777 on the host protease Cathepsin L.
FIG. 2A is a schematic diagram showing that the small molecule Nafamostat has no inhibitory activity against Cathepsin L; FIG. 2B is a schematic diagram showing that the small molecule K777 has no inhibitory activity against TMPRSS2.
FIG. 3 is a schematic diagram of an *in vitro* cell-virus assay using Nafamostat and K777 to inhibit the infection of cells by SARS-CoV-2 Delta.

### DETAILED DESCRIPTION OF THE PREFERRED EMBODIMENT

### Example 1: In vitro enzyme activity inhibition assay

A 384-well plate (brand: PerkinElmer, catalog number: 6007270) was used to dispense TMPRSS2 protein solution (brand: GL Biochem, catalog number: 117075) into 4 rows × 20 columns, with 40 µL per well. Into the first 14 columns of the protein solution, 5 µL of Nafamostat at 14 concentration gradients (initial concentration of 400 nM, 2.5-fold gradient dilution, brand: Selleck, catalog number: S1386) was added as the experimental group. Columns 15 to 20 were added with an equivalent volume of DMSO as a positive control group, and columns 18 to 20 were added with an equivalent volume of buffer as a negative control group. Finally, 5 µL of substrate Boc-Gln-Ala-Arg-AMC (brand: GL Biochem, catalog number: 117075) was added to each well. The 384-well plate was placed in a microplate reader for detection, and the slope of fluorescence intensity increase in the first 2 minutes of the enzymatic reaction was taken as the activity of the protein for subsequent calculation.

The substrate was then replaced with the Cathepsin L-specific substrate Cbz-Phe-Arg-AMC (brand: GL Biochem, catalog number: 974233), and the drug was replaced with K777 (brand: MCE, catalog number: HY-119293), following the same procedure as described above.

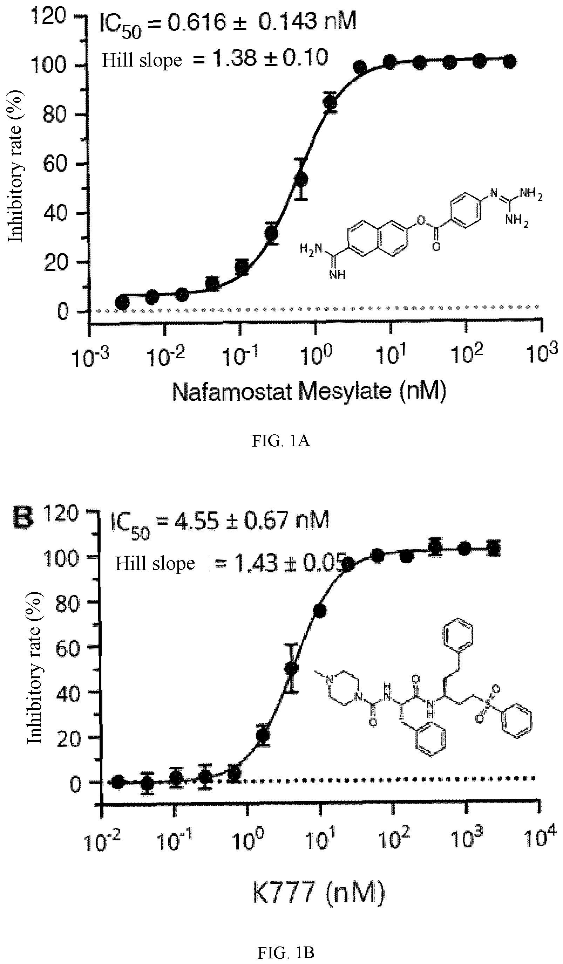

The results of the *in vitro* biochemical enzyme activity inhibition assay demonstrate that Nafamostat exhibits very high inhibitory effects against the serine protease TMPRSS2, with an IC₅₀ value of 0.62 nM (FIG. 1A). K777 exhibits significant inhibitory activity against Cathepsin L, with an IC₅₀ value of 4.55 nM (FIG. 1B).

### Example 2: Cross-validation of in vitro enzyme activity inhibition assay

The procedure was essentially the same as in Example 1, with the only difference being that the drugs and substrates were cross-swapped.

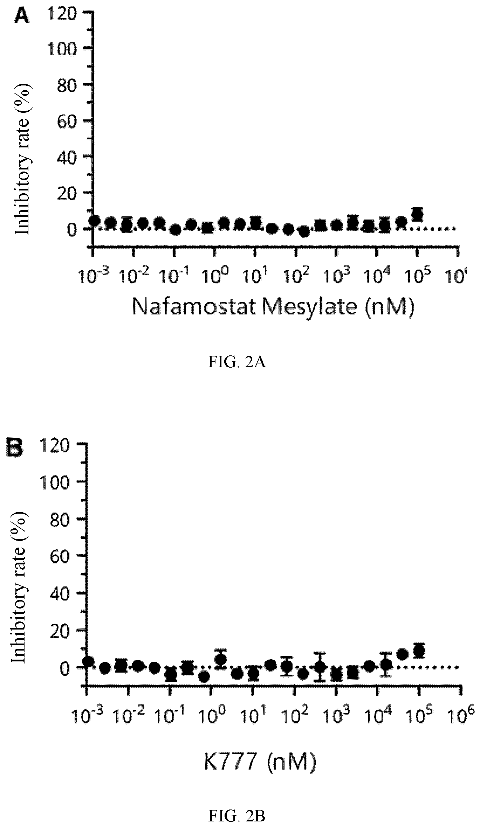

The cross-validation results of the *in vitro* enzyme activity inhibition assay show that Nafamostat exhibits no inhibitory activity against Cathepsin L, and K777 exhibits no inhibitory activity against TMPRSS2 (FIG. 2A and FIG. 2B), thereby confirming the specificity of each drug for its target.

### Example 3: In vitro cell-virus assay

The cytopathic effect method was employed to determine the half-maximal effective concentration (EC₅₀) of the test substances Nafamostat and K777.
(1) On the day prior to the experiment, Calu-3 cells (brand: ATCC, catalog number: HTB-55) were seeded at 2 × 10⁵ cells per well in a 24-well plate (brand: Thermo Fisher, catalog number: 142475);
(2) Nafamostat was serially diluted 5-fold at a maximum concentration of 20 µM, K777 was serially diluted 5-fold at a maximum concentration of 40 µM, and the combination of Nafamostat and K777 was serially diluted 5-fold at a maximum concentration of 20 µM, for a total of 6 or 8 dilutions, with three replicate wells per dilution; Cells were pretreated for 1 hour, and the control group was added with an equivalent volume of DMSO;
(3) After entering the BSL-3 laboratory, SARS-CoV-2 Delta virus suspension (preservation number: Delta-IM2175251-P3-YQ-500 µL, Guangzhou Customs District Technology Center) containing 1 multiplicity of infection (MOI) was added to each well and incubated for 1 hour at 37°C;
(4) The cell culture supernatant was aspirated and discarded, and each well was washed with 500 µL of PBS (brand: Gibco, catalog number: 10099) for three times; Then, the drugs with corresponding concentrations were added, and the cells were incubated at 37°C in a constant-temperature/humidity incubator with 5% CO₂ for 24 hours;
(5) After 24 hours of incubation, cell growth was observed and recorded under a microscope, and the supernatant was collected, and the titer was determined using Vero E6 cells (brand: ATCC, catalog number: CRL-1586);
(6) After 72 hours, the cytopathic rate was read using a Celigo Image Cytometer (Celigo), and the viral titer was calculated using the Reed-Muench method;
(7) EC₅₀ was calculated as follows: 100 × (1 - mean titer of the drug group/mean titer of the DMSO group).

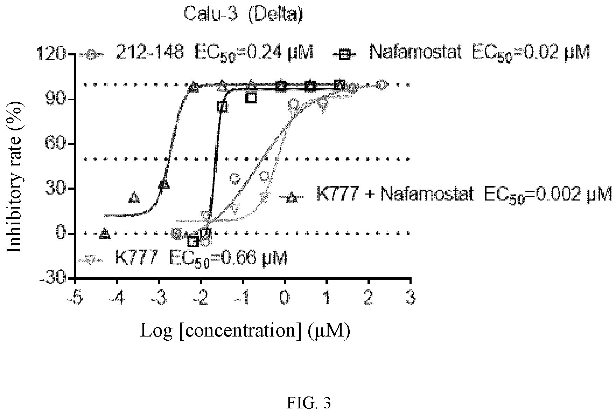

Through the *in vitro* cell-virus assay, it was found that both Nafamostat and K777 could significantly inhibit the infection of SARS-CoV-2 Delta to cells, with EC₅₀ values of 0.02 µM and 0.66 µM, respectively; When the two drugs were used in combination at a molar ratio of 1:1, their antiviral ability was significantly enhanced compared to the use of a single drug, with an EC₅₀ value of 0.002 µM (FIG. 3).

The present disclosure demonstrates that the combined use of Nafamostat and K777, which are inhibitors of the host proteases TMPRSS2 and Cathepsin L, exhibits a 10-fold or more increase in efficacy in anti-SARS-CoV-2 assays, indicating a significant synergistic effect between the two.

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

1. A pharmaceutical combination comprising active ingredients Nafamostat and K777.

2. The pharmaceutical combination according to claim 1, wherein the pharmaceutical combination has a molar ratio of Nafamostat to K777 of 1:0.5 to 1:2.

3. The pharmaceutical combination according to claim 2, wherein the molar ratio is preferably 1:1.

4. The pharmaceutical combination according to claim 2, wherein the pharmaceutical combination further comprises a pharmaceutically acceptable carrier or excipient.

5. The pharmaceutical combination according to claim 1, wherein the pharmaceutical combination has a dosage form selected from one of granules, tablets, pills, capsules, and injections.

6. A use of the pharmaceutical combination according to any one of claims 1 to 5 in the manufacture of a medicament for inhibiting a coronavirus.

7. The use according to claim 6, wherein the coronavirus is SARS-CoV-2, SARS-CoV, MERS-CoV, HCoV-HKU1, HCoV-NL63, HCoV-OC43, or HCoV-229E.

8. The use according to claim 7, wherein the coronavirus is SARS-CoV-2.

9. A use of the pharmaceutical combination according to any one of claims 1 to 5 in the manufacture of a medicament for treating/preventing a disease caused by a coronavirus.

10. The use according to claim 9, wherein the coronavirus is SARS-CoV-2, SARS-CoV, MERS-CoV, HCoV-HKU1, HCoV-NL63, HCoV-OC43, or HCoV-229E.

11. The use according to claim 10, wherein the coronavirus is SARS-CoV-2.

12. A method for treating/preventing a disease caused by a coronavirus, wherein the method comprises administering the pharmaceutical combination according to any one of claims 1 to 5 or a medicament comprising the pharmaceutical combination to a subject in need thereof.

13. The method according to claim 12, wherein the coronavirus is SARS-CoV-2, SARS-CoV, MERS-CoV, HCoV-HKU1, HCoV-NL63, HCoV-OC43, or HCoV-229E.

14. The method according to claim 13, wherein the coronavirus is SARS-CoV-2.

15. A kit set, wherein the kit set comprises a kit A and a kit B, wherein the kit A has a primary active ingredient of Nafamostat, and the kit B has a primary active ingredient of K777.
